# EUROPEAN PATENT APPLICATION

(11) **EP 4 570 160 A1**
(43) Date of publication of application: **18.06.2025**
(21) Application number: 23216068.9
(22) Date of filing: 12.12.2023
(51) Int. Cl.: A61B 3/00, A61B 3/12, G02B 1/00, G02B 21/00, G02B 21/02, G02B 21/08, F21V 8/00

(54) **OPTICAL SYSTEM, IMAGING SYSTEM AND CORRESPONDING METHOD**

(71) Applicant: Leica Instruments (Singapore) Pte Ltd, Singapore 608924 (SG)
(72) Inventor: Zeng, Zhengdong, 608924 Singapore (SG); Schutz, Marco, 608924 Singapore (SG); Leck, Kheng Swee, 608924 Singapore (SG); Kok, Alvin, 608924 Singapore (SG)
(74) Representative: 2SPL Patentanwälte PartG mbB

(57) **Abstract**

The present invention essentially relates to an optical system (100, 200) comprising an imaging flat lens (110) and a light guiding arrangement (120) of flat shape, the optical system (100) having an object side (104) and an image side (102), wherein the light guiding arrangement (120) is configured to receive illumination light (152) and provide the illumination light to the object side (104), wherein the optical system (100) is configured to allow light to be transmitted from the object side (104) via the imaging flat lens (110) to the image side (102).

## Description

### Technical Field

The present invention essentially relates to an optical system comprising an imaging flat lens and a light guiding arrangement of flat shape, to an imaging system comprising such optical system, and to a corresponding method for imaging an object.

### Background

In medical or surgical microscopy or other kind of imaging, e.g., in ophthalmology, optical systems can be used to provide a clear view of a desired object, e.g., a retina of a patient's eye, to a surgeon, including proper illumination.

### Summary

In view of the situation described above, there is a need for improvement in providing a view and illumination of a desired object. According to embodiments of the invention, an optical system, an imaging system and a method with the features of the independent claims are proposed. Advantageous further developments form the subject matter of the dependent claims and of the subsequent description.

An embodiment of the invention relates to an optical system comprising an imaging flat lens and a light guiding arrangement of flat shape. The optical system has an object side and an image side. The light guiding arrangement is configured to receive illumination light and provide the illumination light to the object side. Further, the optical system is configured to allow light to be transmitted from the object side via the imaging flat lens to the image side.

Retinal surgeries, for example, require a clear view of the retina to perform precise operations. However, traditional retinal viewing accessories have several pain points, including a limited field of view, lack of visibility in the posterior of the eye due to no or low illumination, and expensive or bulky retinal viewing systems that can be cost-prohibitive or cumbersome to use. The proposed optical system can overcome these drawbacks and provided a clear view, including proper illumination of an object, e.g., the retina of a patient's eye. Due to the imaging flat lens and the flat shaped light guiding arrangement, the entire optical system is more flexible and thinner than traditional optical systems, and even disposable, while providing a wider field of view and an illumination that can be directed into the periphery of the eye. This can, for example, improve visibility during anterior surgery to observe the anterior chamber angle for glaucoma surgery and for posterior surgery to observe the posterior chamber and retina layer.

Other illumination systems comprise reduction lens and a front lens. In addition, a hole needs to be made into, e.g., the eyeball, to insert a fibre for illimitation, and a holder may be used to fix the fibre in place. In addition, the visibility is often poor with low illumination and the field of view limited due to current optics technology. These disadvantages, i.e., making a hole in the posterior cavity, that the front lens can be bulky and cumbersome and the poor illumination, are overcome with the proposed optical system.

Further, the optical system having an imaging flat lens and a flat light guiding arrangement, is more comfortable to wear during surgery, which can help reduce a patient's discomfort. In addition, the wider field of view provides surgeons with a clearer view of the retina, which can improve surgical outcomes. Further, the light guiding arrangement improves visibility during posterior surgery, which can reduce the risk of complications. The optical system or at least a contact lens thereof can be made reusable or disposable depending on cost.

In an embodiment, the optical system is configured to be in contact with a patient's eye or another object when in use, i.e., it can be placed on the eye during retinal surgery. However, the optical system can also be placed at or within a certain distance from the object or eye, e.g., 5 or 10 mm at maximum; also, the optical system can be placed within a distance between 0 and 10 mm from the object or eye.

In an embodiment, the light guiding arrangement comprises one or multiple illumination flat lenses, wherein the one or the multiple illumination flat lenses are configured to provide the illumination light to the object side. Using flat lenses for illumination allows providing a light guiding arrangement of flat shape.

In an embodiment, the light guiding arrangement comprises multiple illumination flat lenses, wherein at least two peripheral illumination lenses of the multiple illumination flat lenses are arranged at a periphery of the optical system. In this way, the central area of the optical system can be used for imaging or viewing the object, without the light system blocking the view.

In an embodiment, the light guiding arrangement comprises multiple illumination flat lenses and one or multiple light paths, wherein the multiple illumination flat lenses are optically connected to each other via the one or the multiple light paths. In this way, separate (e.g., small) illumination flat lenses can be used, and the illumination light can be provided to each of these flat lenses easily.

In an embodiment, the one or at least two of the multiple light paths, by which the at least two peripheral illumination lenses are optically connected, are arranged in a circular shape. This also helps for the central area of the optical system to be used for imaging or viewing the object, without the light system blocking the view.

In an embodiment, the optical system further comprises an illumination input, wherein the illumination input is configured to receive the illumination light to be provided to the one or the multiple illumination flat lenses. For example, a light fibre or the like can be connected or coupled (optically and/or mechanically) to such illumination input. This allows a quick setting up of an imaging system.

In an embodiment, the light guiding arrangement further comprises a glare light mask, wherein the glare light mask is arranged such that at least a part of glare light, that originates form the light guiding arrangement, is prevented from leaving the optical system at the image side. In this way, proper illumination can be provided which, at the same time, does not or at least not much disturb the view or imaging of the object.

In an embodiment, the glare light mask comprises one or multiple masking parts, each arranged on the one or one of the multiple illumination flat lenses on the imaging side. In this way, any glare can be prevented very effectively. In addition, such (further) masking parts can also be arranged on the light paths and/or the illumination input.

In an embodiment, the imaging flat lens and the light guiding arrangement are arranged next to each other or are integrated into one another. In this way, a compact optical system can be provided.

In an embodiment, the light guiding arrangement is configured to cover less than 20% or less than 10% of an area of the imaging flat lens. In particular, the light guiding arrangement should be as small as possible to prevent the generation of shadows in the image.

Another embodiment of the invention relates to imaging system comprising an optical system in an embodiment as described above. The imaging system further comprises an illumination light source, and at least one of an imaging sensor and an eyepiece. The at least one of an imaging sensor and an eyepiece is arranged at the imaging side of the optical system, and the illumination light source is optically connected to the light guiding arrangement of the optical system, for example, via a light fibre.

In an embodiment, the imaging flat lens is configured for retina imaging, i.e., the imaging flat lens is configured as objective lens of the imaging system for a retina of an eye (where the retina or the eye is the object to be imaged). In an embodiment, the imaging system is configured as ophthalmologic medical imaging system or, in particular, an ophthalmologic surgical imaging system.

Another embodiment of the invention relates to a method for imaging an object, using the optical system or the imaging system in an embodiment as described above. The optical system is placed within a pre-defined distance to the object. This can, for example, mean that the optical system is placed onto the eye of a patient or very close to it.

Further advantages and embodiments of the invention will become apparent from the description and the appended figures.

It should be noted that the previously mentioned features and the features to be further described in the following are usable not only in the respectively indicated combination, but also in further combinations or taken alone, without departing from the scope of the present invention.

### Short description of the Figures

- Fig. 1a: schematically shows an optical system according to an embodiment of the invention;
- Fig. 1b: schematically shows a part of the optical system of Fig. 1a in another view;
- Fig. 1c: schematically shows the optical system of Fig. 1a in another view;
- Fig. 1d: schematically shows the optical system of Fig. 1a in another view;
- Fig. 2: schematically shows an imaging system according to a further embodiment of the invention; and
- Fig. 3: schematically shows a method for imaging an object according to an embodiment of the invention.

### Detailed Description

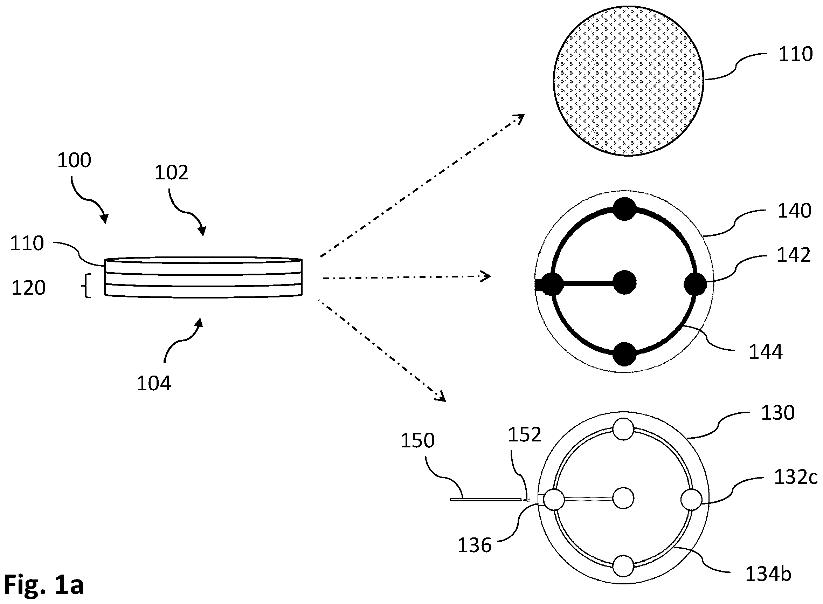

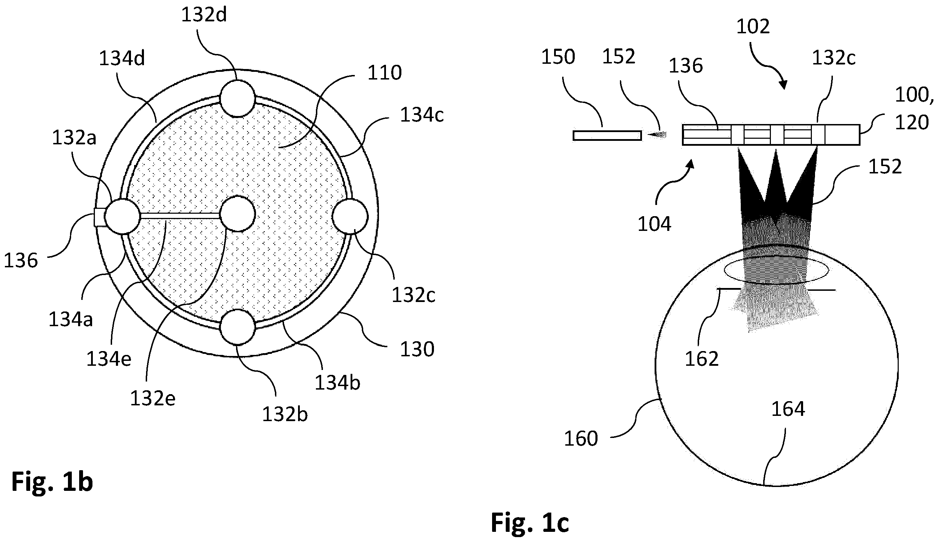

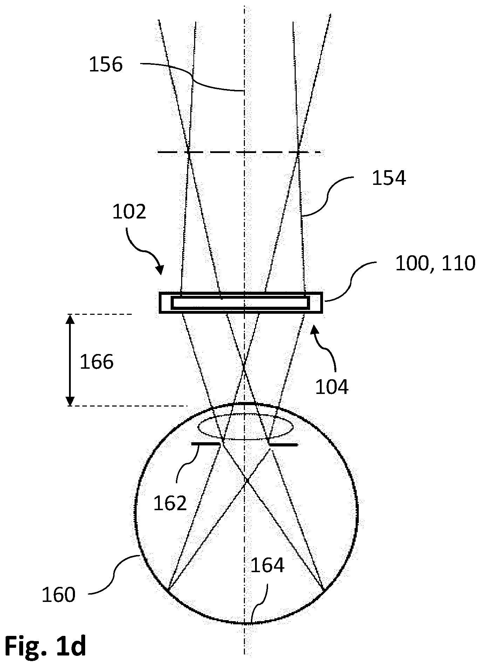

Fig. 1a schematically illustrates an optical system 100 according to an embodiment of the invention. The left side of Fig. 1 shows a side view and the right side shows different parts of the optical system separately in a top view. Fig. 1b illustrates a part of the optical system of Fig. 1a in another view, Fig. 1c illustrates the optical system of Fig. 1a in another view, and Fig. 1d illustrates the optical system of Fig. 1a in even another view. Figs. 1a, 1b, 1c and 1d will be described together in the following.

The optical system 100 comprises an imaging flat lens 110 and a light guiding arrangement 120 of flat shape. The optical system 100 has an object side 104 and an image side 102, shown in the illustration at the left side of Fig. 1a. The light guiding arrangement 120 is configured to receive illumination light 152 and provide the illumination light to the object side 104; this is illustrated in Fig. 1c (showing, in particular, the light guiding arrangement 120 of the optical system 100). Further, the optical system 100 is configured to allow light to be transmitted from the object side 104 via the imaging flat lens 110 to the image side 102.

The optical system 100 can be configured for use with white light or visible light but also for use with, for example, infrared or near-infrared light. Also, the optical system 100 can be configured for use with both and/or even other types of light; this can depend on the desired application.

In an embodiment, the light guiding arrangement 120 comprises one or multiple illumination flat lenses; by means of example, five illumination flat lenses 132a, 132b, 132c, 132d, 132e are shown. The illumination flat lenses are arranged in a layer 130. All reference numerals for illumination flat lenses are shown in Fig. 1b, where Fig. 1b shows the imaging flat lens 110 and layer 130 in combination. The illumination flat lenses are configured to provide the illumination light 152 to the object side. It is noted that also another number of illumination flat lenses can be provided. In addition, by means of example, the illumination flat lenses are of circular shape in cross section, however, another shape could be used.

By means of example, four illumination flat lenses 132a, 132b, 132c, 132d are arranged in at periphery of the optical system 100. In addition, the four illumination flat lenses 132a, 132b, 132c, 132d are uniformly distributed over the periphery, i.e., spaced at angles of 90°. The illumination flat lens 132e is, by means of example, located in the centre of the optical system 100.

In an embodiment, the light guiding arrangement 120 comprises multiple light paths 134a, 134b, 134c, 134d, 134e, wherein the multiple illumination flat lenses 132a, 132b, 132c, 132d, 132e are optically connected to each other via the one or the multiple light paths 134a, 134b, 134c, 134d, 134e. By means of example, light path 134a connects lens 134a to lens 134b, light path 134b connects lens 134b to lens 134c, light path 134c connects lens 134c to lens 134d, light path 134d connects lens 134d to lens 134a, and light path 134e connects lens 134a to lens 134e. The light paths are also arranged in layer 130. All reference numerals for light paths are shown in Fig. 1b.

In an embodiment, the guiding arrangement 120 comprises an illumination input 136, wherein the illumination input 136 is configured to receive the illumination light 152 to be provided to the illumination flat lenses. Such illumination input 136 can be, for example, similar to a light path and be configured to be connected to a light fibre. A light fibre 150 is, by means of example, illustrated in Figs. 1a and 1c, however, without being connected to the illumination input 136.

In this way, illumination light 152 can be provided to the illumination flat lenses of the light guiding arrangement 120, and thus, to the object side 104 as illustrated in Fig. 1c. This allows proper illumination of, e.g., the retina 164 of an eye 160, where the illumination light 152 enters the eye 160 via the iris 162.

In an embodiment, the guiding arrangement 120 comprises a glare light mask 140, wherein the glare light mask 140 is arranged such that at least a part of glare light, that originates from the light guiding arrangement 120, is prevented from leaving the optical system at the image side 102.

In an embodiment, the glare light mask 140 comprises multiple masking parts 142, each arranged on one of the multiple illumination flat lenses on the imaging side 102. In Fig. 1a, only one of five such masking parts 142 is indicated by means of a reference numeral; however, as can be seen from Figs. 1a, 1b, one of those masking parts 142 is provided for each of the multiple illumination flat lenses 132a, 132b, 132c, 132d, 132e.

In an embodiment, the glare light mask 140 comprises multiple additional masking parts 144, each arranged on one of the multiple light paths and also the illumination input on the imaging side 102. In Fig. 1a, only one of such additional masking parts 144 is indicated by means of a reference numeral; however, as can be seen from Figs. 1a, 1b, one of those additional masking parts 144 is provided for each of the multiple light paths 134a, 134b, 134c, 134d, 134e and the illumination input 136.

The multiple masking parts 142 and the additional masking parts 144 are all arranged in a thin layer. Thus, the guiding arrangement 120 comprises two layers, a layer 130 comprising the illumination flat lenses and the corresponding light paths and the illumination input, and the glare light mask 140 with its masking parts as another layer. This results in a guiding arrangement 120 of flat shape. In combination with the imaging flat lens 110, which is also flat and can be considered as a (thin) layer, the optical system 100 comprises three layers. In addition, this shows that the imaging flat lens 110 and the light guiding arrangement 120 can be arranged next to each other or even be integrated into one another.

While, for explanation purposes, five illumination flat lenses and corresponding light paths and masking parts of the glare mask are shown, the number and the size and the form of the illumination flat lenses and corresponding light path can differ and can be chosen as required. For example, only one or more larger illumination flat lenses can be provided. Also, more than five illumination flat lenses can be provided, for example, eight or ten or twelve illumination flat lenses; all of them can, for example, be distributed at the periphery of the optical system.

It is noted that the light guiding arrangement 120 should be configured to cover an area as small as possible from the imaging flat lens 110, for example less than 20% or less than 10% or less than 5%. In this way, viewing or imaging of the object is as good as possible, while also illumination is still good.

Fig. 1d illustrates the optical system 100, in particular the imaging flat lens 110, and the eye 160, similar to Fig. 1c. However, whereas Fig. 1c illustrates the illumination of the retina 164 with the light guiding arrangement 120 and illumination light 152, Fig. 1d illustrates the imaging of the retina 164, where an imaging light beam 154 leaves the eye 160 via the iris 162, enters the imaging flat lens 110 at the object side 104 and leaves the imaging flat lens 110 at the image side 102. The imaging light beam 154 can follow the optical axis 156, for example, to a sensor of an imaging system, for example.

Due to the glare mask 140, glare light is prevented from entering the imaging path, i.e., the imaging light beam at the image side, and thus the image will not be degraded.

The optical system 100 can be located close to the eye 160, for example. A distance 166 (see Fig. 1d, also valid for Fig. 1c) between the eye 160 and the optical system 100 can be less than 10 cm or less than 5 cm, for example. Further, the optical system 100 could also be located at the eye 160 or its surface, i.e., the distance 166 could also be zero.

A flat lens, in general, can be a lens whose flat shape allows it to provide distortion-free-imaging, potentially with arbitrarily-large apertures. Flat lenses can require a refractive index close to -1 over a broad angular range, however flat lenses based on metasurface can also be used. A metasurface or electromagnetic metasurface refers to a kind of artificial sheet material with sub-wavelength thickness. Metasurfaces can be either structured or unstructured with subwavelength-scaled patterns in the horizontal dimensions. A metasurface can be an electromagnetic structure engineered on subwavelength scales to elicit tailored polarization responses. Metamaterials can, for example, image infrared, visible, and also ultraviolet wavelengths.

A thickness of such flat lens (this is for the imaging flat lens and/or for the illumination flat lenses), along the optical path, can be less than 200 µm or less than 50 µm or less than 20 µm.

In contrast to flat lenses, traditional curved glass lenses can bend light coming from many angles to end up at the same focal point on a piece of photographic film or an electronic sensor. Light captured at the very edges of a curved glass lens does not line up correctly with the rest of the light, creating a fuzzy image at the edge of the frame. To correct this, lenses use extra pieces of glass, adding bulk, complexity, and mass.

Thus, with the proposed use of flat lenses for imaging and illumination, a very thin optical system can be provided.

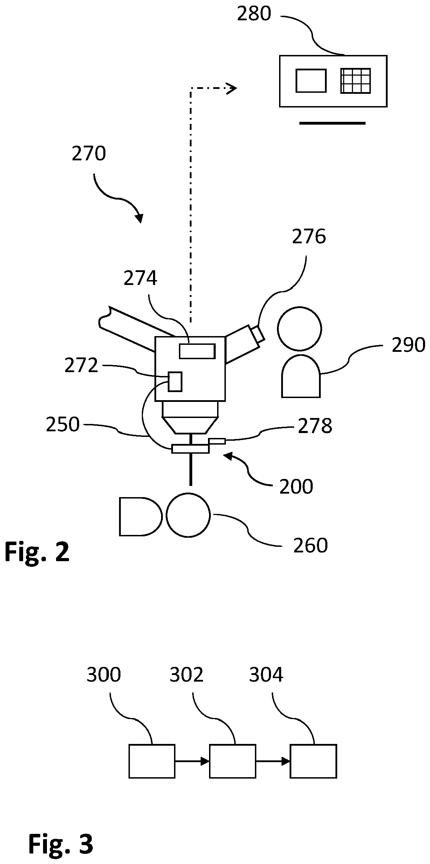

Fig. 2 schematically illustrates an imaging system 270 according to an embodiment of the invention. The imaging system comprise an optical system 200, an illumination light source 272, and, by means of example, an imaging sensor 274 and an eyepiece 276. optical system 200 can comprise or can be configured as the optical system 100 shown in Figs. 1a to 1d. Both, the imaging sensor 274 and an eyepiece 276 are arranged at the imaging side of the optical system 200. The illumination light source 272 is optically connected, by means of example via a light fiber 250, to the light guiding arrangement of the optical system; this can correspond to the situation shown for light fiber 150 and optical system 100 in Figs. 1a, 1c.

By means of example, the imaging system 200 is configured as an ophthalmologic medical imaging system, in particular, a surgical microscope for ophthalmologic or retinal surgeries. The optical system 200 is, in an embodiment, configured as an objective lens of the imaging system 100 for imaging the retina of an eye. By means of example, a patient 260 is shown, who's eye is to be viewed or imaged as described with respect to Figs. 1a to 1d. The optical system 200 can, for example, be held in place by means of a holder 278 of the imaging system 270; the holder 278 can, for example, be fixed to a microscope body or the like.

The imaging system 200 can further comprise, for example, a display 280, on which images produce by the imaging sensor 274 are shown, e.g., for the surgeon 290 or others.

Fig. 3 schematically illustrates a method for imaging an object, using an optical system, e.g., as described with respect to Figs. 1a to 1d or an imaging system described with respect to Fig. 2. By means of example, the method comprises imaging the retina of an eye of a patient.

In step 300, the optical system is placed within a pre-defined distance to the eye; this can comprise, for example, placing the optical system such that it touches the eye of the patient. In step 302, the imaging system can be operated to provided illumination light to the optical system and, via the optical system, to the eye and the retina. In step 304, light reflected form the retina is imaged using the imaging sensor. In addition, or alternatively, a surgeon can view the retina via the eyepiece.

Whereas traditional optical systems typically have a lack of visibility in the posterior of the eye due to no or low illumination, the light guiding arrangement as described herein can be incorporated into the optical system (the lens), using, e.g., a small, lightweight light fibre or fibre optics, that attaches to the side of the lens, and with the other end connected to an illumination light source (see Fig. 2). The light will be directed into the periphery of the eye during posterior surgery using a prism in the contact lens. Various light wavelengths can be transmitted including white light and near infrared light.

Additionally, traditional retinal viewing systems can be expensive or bulky, making them cost-prohibitive or cumbersome to use. The imaging flat lens described herein (contact lens) made from flat lens optics is thinner, more flexible, and disposable, making it easier to use during surgery. The light guiding arrangement (illumination module) can be easily attached and removed as needed, or made disposable, reducing the need for expensive and bulky retinal viewing systems.

Thus, the proposed optical system provides a significant improvement over traditional retinal viewing accessory. By addressing pain points such as limited field of view, lack of visibility in the posterior of the eye, and cost-prohibitive and cumbersome retinal viewing systems, the proposed optical system made from flat lens optics and an illumination module has the potential to improve surgical outcomes and reduce patient discomfort.

As used herein the term "and/or" includes any and all combinations of one or more of the associated listed items and may be abbreviated as "f".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100, 200: optical system
- 102: object side
- 104: image side
- 110: imaging flat lens
- 120: light guide arrangement
- 130: layer
- 132a, 132b, 132c, 132d, 132e: illumination flat lens
- 134a, 134b, 134c, 134d, 134e: light path
- 136: illumination input
- 140: glare mask
- 142: masking part
- 144: additional masking part
- 150, 250: light fibre
- 152: illumination light
- 154: imaging light beam
- 156: optical path
- 160: eye
- 162: iris
- 164: retina

- 260: patient
- 270: imaging system
- 272: imaging sensor
- 274: eyepiece
- 278: holder
- 280: display
- 290: surgeon

- 300, 302, 304: method steps

## Claims

1. An optical system (100, 200) comprising an imaging flat lens (110) and a light guiding arrangement (120) of flat shape, the optical system (100) having an object side (104) and an image side (102),
wherein the light guiding arrangement (120) is configured to receive illumination light (152) and provide the illumination light to the object side (104),
wherein the optical system (100) is configured to allow light to be transmitted from the object side (104) via the imaging flat lens (110) to the image side (102).

2. The optical system (100, 200) of claim 1, wherein the light guiding arrangement (120) comprises one or multiple illumination flat lenses (132a, 132b, 132c, 132d, 132e), wherein the one or the multiple illumination flat lenses (132) are configured to provide the illumination light (152) to the object side (104).

3. The optical system (100, 200) of claim 2, wherein the light guiding arrangement (120) comprises multiple illumination flat lenses (132a, 132b, 132c, 132d, 132e), wherein at least two peripheral illumination lenses of the multiple illumination flat lenses are arranged at a periphery of the optical system (100).

4. The optical system (100, 200) of claim 2 or 3, wherein the light guiding arrangement (120) comprises multiple illumination flat lenses (132a, 132b, 132c, 132d, 132e) and one or multiple light paths (134a, 134b, 134c, 134d, 134e), wherein the multiple illumination flat lenses (132a, 132b, 132c, 132d, 132e) are optically connected to each other via the one or the multiple light paths (134a, 134b, 134c, 134d, 134e).

5. The optical system (100, 200) of claims 3 and 4, wherein the one or at least two of the multiple light paths (134a, 134b, 134c, 134d, 134e), by which the at least two peripheral illumination lenses are optically connected, are arranged in a circular shape.

6. The optical system (100) of any one of claims 2 to 5, further comprising an illumination input (136), wherein the illumination input (136) is configured to receive the illumination light (152) to be provided to the one or the multiple illumination flat lenses (134a, 134b, 134c, 134d, 134e).

7. The optical system (100, 200) of any one of the preceding claims, wherein the light guiding arrangement (120) further comprises a glare light mask (140), wherein the glare light mask is arranged such that at least a part of glare light, that originates from the light guiding arrangement (120), is prevented from leaving the optical system at the image side (102).

8. The optical system (100, 200) of any one of claims 2 to 6, and of claim 7, wherein the glare light mask (140) comprises one or multiple masking parts (142), each arranged on the one or one of the multiple illumination flat lenses (132a, 132b, 132c, 132d, 132e) on the imaging side (102).

9. The optical system (100, 200) of any one of the preceding claims, wherein the imaging flat lens (110) and the light guiding arrangement (120) are arranged next to each other or are integrated into one another.

10. The optical system (100, 200) of any one of the preceding claims, wherein the light guiding arrangement (120) is configured to cover less than 20% or less than 10% of an area of the imaging flat lens (110).

11. An imaging system (270) comprising the optical system (100, 200) of any one of the preceding claims, an illumination light source (272), and at least one of an imaging sensor (274) and an eyepiece (276),
wherein the at least one of an imaging sensor and an eyepiece is arranged at the imaging side of the optical system, and
wherein the illumination light source is optically connected to the light guiding arrangement of the optical system.

12. The imaging system (270) of claim 11, configured as ophthalmologic medical imaging system.

13. The imaging system (270) of claim 11 or 12, wherein the imaging flat lens (110) is configured as an objective lens for a retina of an eye.

14. A method for imaging an object, using the optical system of any one of claims 1 to 10 or the imaging system of claim 11 or 12, wherein the optical system is placed within a pre-defined distance to the object.

15. The method of claim 14, wherein the object comprises or is a retina (164) of an eye (160) of a patient, and wherein the optical system is placed outside the eye.
